(19) Europäisches Patentamt
European Patent Office
Office européen des brevets

(11) Numéro de publication : **0 677 525 A1**

(12) # DEMANDE DE BREVET EUROPEEN

(21) Numéro de dépôt : **95400775.3**

(51) Int. Cl.[6] : **C07F 5/02,** C07K 5/06

(22) Date de dépôt : **07.04.95**

(30) Priorité : **12.04.94 FR 9404287**

(43) Date de publication de la demande :
**18.10.95 Bulletin 95/42**

(84) Etats contractants désignés :
**AT BE CH DE DK ES FR GB GR IE IT LI LU NL PT SE**

(71) Demandeur : **SYNTHELABO**
**22, Avenue Galilée**
**F-92350 Le Plessis Robinson (FR)**

(72) Inventeur : **Mallart, Sergio**
**17ter avenue du Maréchal Joffre**
**F-91400 Orsay (FR)**
Inventeur : **Lassalle, Gilbert**
**53 avenue Victor Hugo**
**F-92140 Clamart (FR)**
Inventeur : **Purcell, Thomas Andrew**
**47bis rue de la Millière Les Mesnuls**
**F-78490 Montfort l'Amaury (FR)**
Inventeur : **Muller, Jean Claude**
**4 avenue de l'Espérance**
**F-91390 Morsang sur Orge (FR)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

(54) **Dérivés d'acides aminoboroniques, leur préparation et leur utilisation comme intermédiaires de synthèse.**

(57) Composés de formule (I)

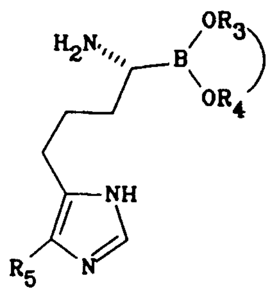

(I)

dans laquelle
$R_3$ et $R_4$ représentent ensemble le résidu d'un composé dihydroxylé tel que par exemple le butane-2,3-diol, le 2,3-diméthylbutane-2,3-diol ou le (1α,3α,5α)-2,6,6-triméthylbicyclo[3.1.1]heptane-2,3-diol[(+)-α-pinanediol] et $R_5$ représente un atome d'hydrogène ou un groupe ($C_1$-$C_4$)alkyle sous forme d'isomères optiques ou géométriques, purs ou sous forme de mélanges, ainsi que leurs sels.
Intermédiaires de synthèse.

EP 0 677 525 A1

Jouve, 18, rue Saint-Denis, 75001 PARIS

La présente invention a pour objet des dérivés d'acides aminoboroniques, leur préparation et leur utilisation comme intermédiaires de synthèse.

Les composés de l'invention répondent à la formule (I)

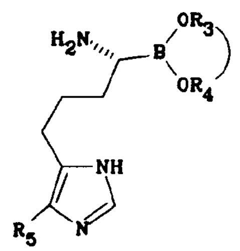

(I)

dans laquelle
$R_3$ et $R_4$ représentent ensemble le résidu d'un composé dihydroxylé tel que par exemple le butane-2,3-diol, le 2,3-diméthylbutane-2,3-diol ou le (1α, 3α, 5α)-2,6,6-triméthylbicyclo[3.1.1]heptane-2,3-diol [(+)-α-pinanediol] et $R_5$ représente un atome d'hydrogène ou un groupe ($C_1$-$C_4$)alkyle.

Les composés de l'invention peuvent exister sous forme d'isomères optiques ou géométriques, purs ou sous forme de mélanges. Ils peuvent exister sous forme de bases libres ou de sels.

Les composés de l'invention peuvent être synthétisés selon le schéma 1.
On fait réagir le [3*aS*-(3*a*α, 4β, 6β, 7*a*α)]-2-(3-bromopropyl)-3a,5,5-triméthylhexahydro-4,6-méthano-1,3,2-benzodioxaborole de formule (IV) avec de l'iodure de sodium dans un solvant tel que par exemple l'acétone pour obtenir le [3*aS*-(3*a*α, 4β, 6β, 7*a*α)]-2-(3-iodopropyl)-3*a*,5,5-triméthylhexahydro-4,6 méthano-1,3,2-benzodioxaborole de formule (V) que l'on condense avec un composé de formule (VI) (dans laquelle $R_5$ représente un atome d'hydrogène ou un groupe ($C_1$-$C_4$)alkyle), pour obtenir le composé de formule (VII); la réaction est réalisée dans un solvant tel que le tétrahydrofurane, à une température comprise entre -78 et +20 °C. Ensuite, selon un procédé analogue à celui décrit par Mattheson,

## Schéma 1

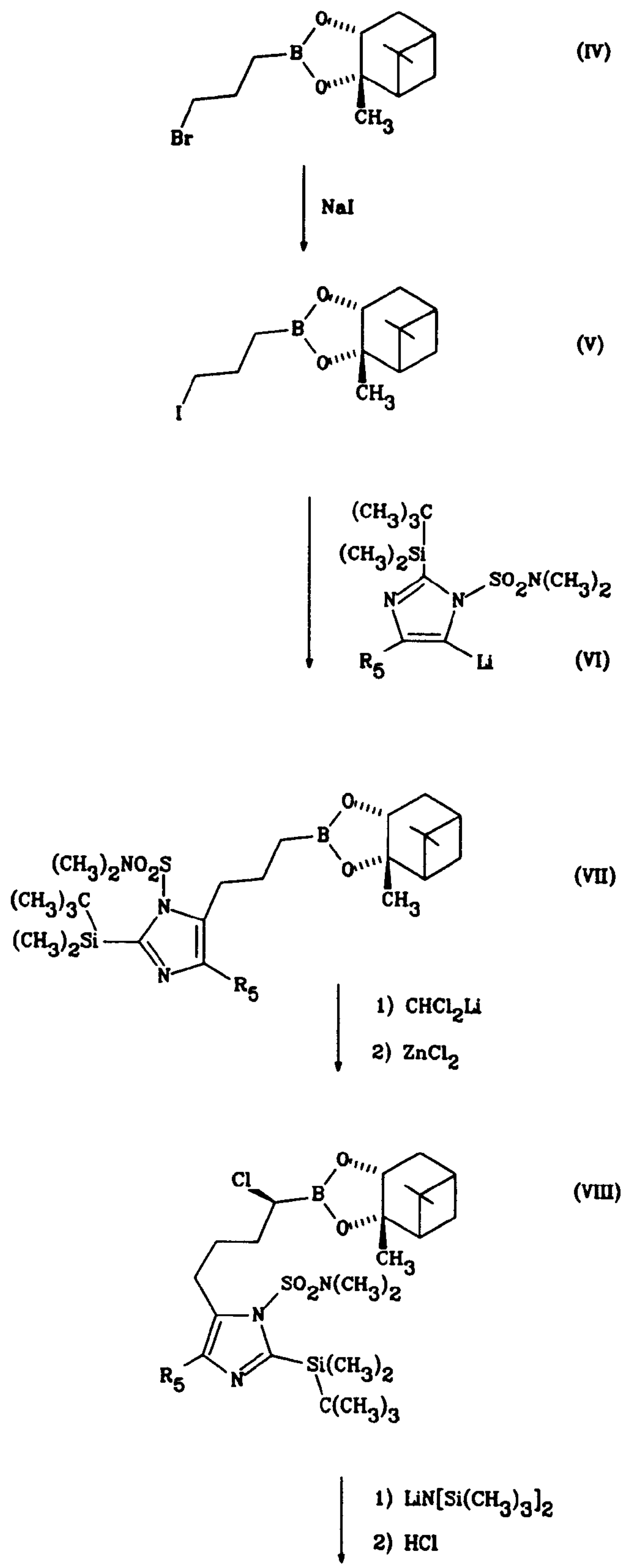


(IV)

(V)

(VI)

(VII)

(VIII)

**Schéma 1 (suite)**

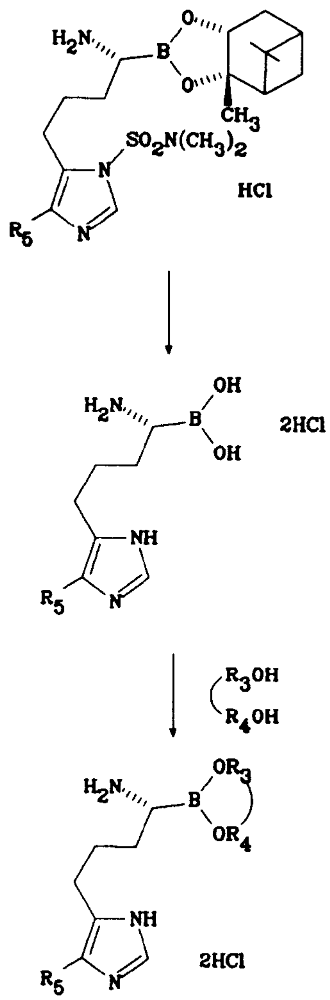


(IX)

(X)

(XI)

(I)

Organometallics, (1984), 3, 614, on fait réagir le composé de formule (VII) avec du dichlorométhyllithium, en présence de chlorure de zinc, dans un solvant tel que le tétrahydrofurane, à une température comprise entre -100 et +20 °C, pour obtenir le composé de formule (VIII) que l'on fait réagir avec du bis(triméthylsilyl)amidure de lithium ; la réaction est réalisée dans un solvant tel que le tétrahydrofurane à une température comprise entre -78 et +20 °C. Ensuite, on traite le composé ainsi obtenu avec de l'acide chlorhydrique dans un solvant tel que par exemple le dioxane et on obtient le chlorhydrate de formule (IX) que l'on hydrolyse pour former le dichlorhydrate de l'acide (*R*)-α-amino-butaneboronique de formule (X) puis on fait réagir l'acide ainsi obtenu avec un diol de formule (XI) dans laquelle $R_3$ et $R_4$ représentent ensemble le résidu d'un composé dihydroxylé tel que défini précédemment.

Les composés de départ sont disponibles dans le commerce ou décrits dans la littérature ou peuvent être préparés selon des méthodes qui y sont décrites ou qui sont connues de l'homme du métier.

Ainsi, le [3a*S*-(3aα, 4β, 6β, 7aα)]-2-(3-bromopropyl)-3*a*,5,5 triméthylhexahydro-4,6-méthano-1,3,2-benzodioxaborole est décrit dans le brevet européen no 0293881.

Le 2-[(1,1-diméthyléthyl)diméthylsilyl]-*N*,*N*-diméthyl-1*H*-imidazole-1-sulfonamide est décrit par Ngochindo dans J. Chem. Soc. Perkin Trans. (1990), 1, 1645 et le 2-[(1,1-diméthyléthyl)diméthylsilyl]-*N*,*N*,4-triméthyl-1*H*-imidazole-1-sulfonamide est préparé par une méthode analogue.

Les exemples suivants illustrent la préparation de quelques composés conformément à l'invention.

Les microanalyses élémentaires et les spectres IR et RMN confirment la structure des composés obtenus.

Exemple 1

chlorhydrate de [3a*S*-[2(*R*), 3aα, 4β, 6β, 7aα]]-α-(3*a*,5,5-triméthylhexahydro-4,6-méthano-1,3,2-benzodioxaborol-2-yl)-1*H*-imidazole-4(5)-butanamine (2:1)

1.1. [3*aS*-(3*a*α, 4β, 6β, 7*a*α)]-2-(3-iodopropyl)-3*a*,5,5-triméthylhexahydro-4,6-méthano-1,3,2-benzodioxaborole

On porte à la température de reflux pendant 24 heures, une solution de 37 g (122 mmoles) de [3*aS*-(3*a*α, 4β, 6β, 7*a*α)]-2-(3-bromopropyl)-3*a*,5,5-triméthylhexahydro-4,6-méthano-1,3,2-benzodioxaborole et de 72,7 g (488 mmoles) d'iodure de sodium dans 500 ml d'acétone. On évapore le solvant et on reprend le résidu dans un mélange de 500 ml d'éther et de 100 ml d'eau contenant 1 g de sulfite de sodium. On sèche la phase organique sur du sulfate de magnésium, on filtre et on évapore.

On obtient 40 g de produit que l'on utilise directement dans l'étape suivante.

Rendement = 95 %

1.2. [3*aS*-(3*a*α, 4β, 6β, 7*a*α)]-2-[(1,1-diméthyléthyl)diméthylsilyl]-*N*,*N*-diméthyl-4(5)-[3-(3*a*,5,5-triméthylhexahydro-4,6-méthano-1,3,2-benzodioxaborol-2-yl)propyl]-1*H*-imidazole-1-sulfonamide

On dissout 70,5 g (244 mmoles) de 2-[(1,1-diméthyléthyl)diméthylsilyl]-*N*,*N*-diméthyl-1*H*-imidazole-1-sulfonamide dans 250 ml de tétrahydrofurane. On refroidit le milieu réactionnel à -78 °C et on ajoute 152 ml (244 mmoles) d'une solution 1,6 M de n-butyllithium dans l'hexane. On laisse sous agitation pendant 1 heure à -78 °C puis on ajoute 40 g (115 mmoles) de [3*aS*-(3*a*α, 4β, 6β, 7*a*α)]-2-(3-iodopropyl)-3*a*,5,5-triméthylhexahydro-4,6-méthano-1,3,2-benzodioxaborole en solution dans 100 ml de tétrahydrofurane. On agite le milieu réactionnel entre -78 °C et +20 °C pendant 1 heure puis à 20 °C pendant 2 heures. On verse le milieu réactionnel sur 350 ml d'un mélange glace-eau contenant 14,5 g (121 mmoles) d'hydrogénosulfate de sodium. On extrait la phase aqueuse par 3 fois 100 ml d'éther, on réunit les phases éthérées, on les sèche sur sulfate de magnésium, on filtre et on évapore. On purifie le résidu par chromatographie sur colonne de gel de silice en éluant par une solution d'acétate d'éthyle à 20 % dans l'hexane.

On obtient 45 g de produit.

Rendement = 73 %

$[\alpha]_D^{20}$ = +12,5 ° (c = 1,9; chloroforme)

1.3. [3*aS*-[2(*S*), 3*a*α, 4β, 6β, 7*a*α]]-4(5)-[4-chloro-4-(3*a*,5,5-triméthylhexahydro-4,6-méthano-1,3,2-benzodioxaborol-2-yl)butyl]-2-[(1,1-diméthyléthyl)diméthylsilyl]-*N*,*N*-diméthyl-1*H*-imidazole-1-sulfonamide

On refroidit une solution de 8,3 g (98 mmoles) de dichlorométhane dans 100 ml de tétrahydrofurane à -100 °C. On ajoute 39,1 ml (98 mmoles) d'une solution 2,5 M de n-butyllithium dans l'hexane. On laisse pendant 15 minutes à cette température puis on ajoute 45 g (89 mmoles) de [3*aS*-(3*a*α, 4β, 6β, 7*a*α)]-2-[(1,1-diméthyléthyl)diméthylsilyl]-*N*,*N*-diméthyl-4(5)-[3-(3*a*,5,5-triméthylhexahydro-4,6-méthano-1,3,2-benzodioxaborol-2-yl)propyl]-1*H*-imidazole-1-sulfonamide, en solution dans 50 ml de tétrahydrofurane. On laisse le mélange réactionnel pendant 15 minutes à -100 °C et on ajoute 9,8 g (70 mmoles) d'une solution de chlorure de zinc dans 50 ml de tétrahydrofurane. On laisse revenir à +20 °C pendant 16 heures. On évapore sous vide et on reprend le résidu dans un mélange de 200 ml de dichlorométhane et de 50 ml d'eau. On sépare les phases et on extrait la phase aqueuse avec 100 ml de dichlorométhane. On rassemble les phases organiques, on les sèche sur du sulfate de magnésium, on les filtre et on évapore. On purifie le résidu coloré obtenu, par chromatographie sur colonne de gel de silice en éluant par un mélange acétate d'éthyle:hexane (20:80).

On obtient 40 g de produit sous forme d'une huile incolore.

Rendement = 80 %

$[\alpha]_D^{20}$ = +15,9 ° (c = 2,65; chloroforme)

1.4. chlorhydrate de [3*aS*-[2(R), 3*a*α, 4β, 6β, 7*a*α]]-4(5)-[4-amino-4-(3*a*,5,5-triméthylhexahydro-4,6-méthano-1,3,2-benzodioxaborol-2-yl)butyl]-*N*,*N*-diméthyl-1*H*-imidazole-1-sulfonamide (1:1)

On met en solution 12,6 g (78 mmoles) de 1,1,1,3,3,3-hexaméthyldisilazane, dans 80 ml de tétrahydrofurane et on ajoute 31 ml (78 mmoles) d'une solution 2,5 M de n-butyllithium dans l'hexane. On laisse pendant 1 heure à -78 °C et on ajoute 40 g (71 mmoles) de [3*aS*-[2(*S*), 3*a*α, 4β, 6β, 7*a*α]]-4(5)-[4-chloro-4-(3*a*,5,5-triméthylhexahydro-4,6-méthano-1,3,2-benzodioxaborol-2-yl)butyl]-2-[(1,1-diméthyléthyl)diméthylsilyl]-*N*,*N*-diméthyl-1*H*-imidazole-1-sulfonamide, en solution dans 80 ml de tétrahydrofurane. On laisse sous agitation pendant 1 heure à -78 °C et pendant 16 heures à +20 °C. On refroidit le milieu réactionnel à -78 °C, on ajoute 78 ml (312 mmoles) d'une solution 4 N d'acide chlorhydrique dans le dioxane et on laisse sous agitation, pendant 1 heure à -78 °C et pendant 2 heures à +20 °C. On évapore sous vide et on reprend le résidu dans 200 ml de chloroforme. On filtre et on évapore.

On obtient 32 g de produit sous forme d'une huile que l'on triture dans l'éther.

On obtient le produit sous forme d'un solide.

Rendement = 89 %

Point de fusion = 90-92 °C

$[\alpha]_D^{20}$ = +11 ° (c=1 ; méthanol)

1.5. chlorhydrate de [3*aS*-[2(*R*), 3*a*α, 4β, 6β, 7*a*α]]-α-(3*a*,5,5-triméthylhexahydro-4,6-méthano-1,3,2-benzodioxaborol-2-yl)-1*H*-imidazole-4(5)-butanamine (2:1)

On porte au reflux pendant 3 heures, 32 g (70 mmoles) de chlorhydrate de [3*aS*-[2(*R*), 3*a*α, 4β, 6β, 7*a*α]]-4(5)-[4-amino-4-(3*a*,5,5-triméthylhexahydro-4,6-méthano-1,3,2-benzodioxaborol-2-yl)butyl]-*N*,*N*-diméthyl-1*H*-imidazole-1-sulfonamide, en solution dans 200 ml d'acide chlorhydrique 4 N. On extrait la solution par 4 fois 100 ml d'éther et on évapore à sec. On reprend le résidu par 100 ml de méthanol et on ajoute 11,9 g (70 mmoles) de [1*R*-(1α, 2α, 3α, 5α)]-2,6,6-triméthylbicyclo[3.1.1]heptane-2,3-diol. On laisse sous agitation pendant 16 heures à 20 °C et on évapore à sec.

On obtient 27 g de produit sous forme d'une huile que l'on triture dans l'éther.

Point de fusion = 75-80 °C

Exemple 2

chlorhydrate de la [3a*S*[2*R*,3aα,4β,6β,7aα]]-5-méthyl-α-(3a,5,5-triméthylhexahydro-4,6-méthano-1,3,2-benzodioxaborol-2-yl)-1*H*-imidazole-4-butanamine (2:1)

On le prépare selon la méthode décrite dans l'exemple 1 à partir du 2-[(1,1-diméthyléthyl)diméthylsilyl]-*N*,*N*,4-triméthyl-1*H*-imidazole-1-sulfonamide.

On obtient le produit sous forme d'un solide coloré.

Point de fusion = 70-75 °C.

Les composés selon l'invention sont utiles comme intermédiaires pour la synthèse de composés de formule (1)

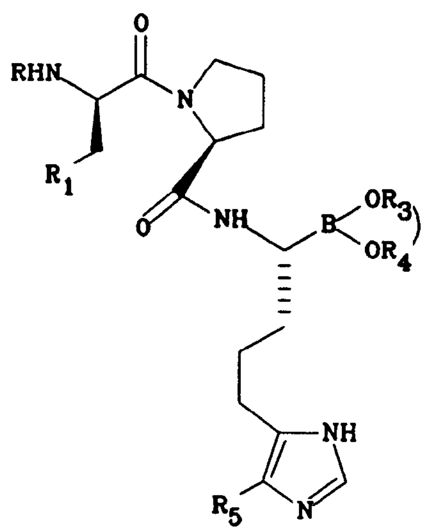

(1)

dans laquelle

R représente soit un atome d'hydrogène, soit un groupe choisi parmi les groupes $(C_1\text{-}C_4)$alkyle droit ou ramifié, -COR' où R' est un groupe $(C_1\text{-}C_4)$alkyle droit ou ramifié et $-(CH_2)_nCO_2R''$ où R'' est atome d'hydrogène ou un groupe $(C_1\text{-}C_4)$alkyle droit ou ramifié ou un groupe phénylméthyle et n = 0 à 2,

$R_1$ représente soit un groupe phényle, soit un groupe naphtalén-1-yle, soit un groupe naphtalén-2-yle, soit un groupe cyclohexyle,

$R_3$ et $R_4$ représentent ensemble le résidu d'un composé dihydroxylé tel que par exemple le butane-2,3-diol, le 2,3-diméthylbutane-2,3-diol ou le (1α, 3α, 5α)-2,6,6-triméthylbicyclo[3.1.1]heptane-2,3-diol [(+)-α-pinanediol] et

$R_5$ représente un atome d'hydrogène ou un groupe $(C_1\text{-}C_4)$alkyle.

On fait réagir un composé de formule (1) selon l'invention avec une forme activée d'un dipeptide de formule (XIII)

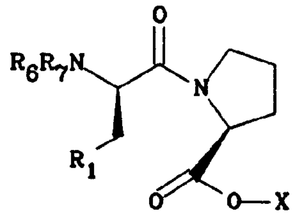

(XIII)

(dans laquelle $R_1$ est tel que défini dans la revendication 1, $R_6$ représente soit un atome d'hydrogène quand $R_7$ représente un groupe $-CO(C_1-C_4)$alkyle droit ou ramifié, soit un groupe $-CO_2(C_1-C_4)$alkyle droit ou ramifié quand $R_7$ représente un atome d'hydrogène ou un groupe $(C_1-C_4)$alkyle droit ou ramifié et X représente soit le groupe pyrrolidin-1-yl-2,5-dione, soit le groupe 2-méthyl-propyloxycarbonyle)

Les exemples 3 et 4 qui suivent illustrent cette synthèse sans la limiter.

Exemple 3

chlorhydrate de [3a*S*-[2(*R*), 3aα, 4β, 6β, 7aα]]-*N*-acétyl-*D*-phénylalanyl-*N*-[4-(1*H*-imidazol-4(5)-yl)-1-(3a,5,5-triméthylhexahydro-4,6-méthano-1,3,2-benzodioxaborol-2-yl)butyl]-*L*-prolinamide (1:1)

3.1. ester de la *N*-acétyl-*D*-phénylalanyl-*L*-proline avec la 1-hydroxypyrrolidine-2,5-dione

On suspend 6 g (20 mmoles) de *N*-acétyl-*D*-phénylalanyl-*L*-proline dans un mélange de 100 ml d'acétate d'éthyle et 5 ml de diméthylformamide. On ajoute 2,53 g (22 mmoles) de 1-hydroxypyrrolidine-2,5-dione et on refroidit à 0 °C. On ajoute ensuite, par petites portions, 4,53 g (22 mmoles) de 1,3-dicyclohexylcarbodiimide sous forme solide. On agite pendant 20 heures à 20 °C et on filtre la suspension. On lave successivement le filtrat avec 20 ml d'une solution d'hydrogénocarbonate de sodium à 5 % puis avec une solution saturée en chlorure de sodium et on sèche sur sulfate de magnésium. On triture le résidu obtenu dans l'éther.

On obtient 8 g de produit vitreux que l'on utilise tel quel dans l'étape suivante.

3.2. chlorhydrate de [3a*S*-[2(*R*), 3aα, 4β, 6β, 7aα]]-*N*-acétyl-*D*-phénylalanyl-*N*-[4-(1*H*-imidazol-4(5)-yl)-1-(3a,5,5-triméthylhexahydro-4,6-méthano-1,3,2-benzodioxaborol-2-yl)butyl]-*L*-prolinamide (1:1)

On solubilise 2,4 g (6,4 mmoles) de chlorhydrate de [3a*S*-[2(*R*), 3aα, 4β, 6β, 7aα]]-α-(3a,5,5-triméthylhexahydro-4,6-méthano-1,3,2-benzodioxaborol-2-yl)-1*H*-imidazole-4(5)-butanamine dans 20 ml de dichlorométhane, on ajoute 2,5 g (6,4 mmoles) de l'ester de la *N*-acétyl-*D*-phénylalanyl-*L*-proline avec la 1-hydroxypyrrolidine-2,5-dione et on refroidit le mélange à -30 °C. On ajoute, goutte à goutte, 3,6 ml (25,6 mmoles) de triéthylamine et on agite pendant 2 heures entre -30 °C et +20 °C puis pendant 2 heures à +20 °C. On ajoute ensuite 20 ml d'une solution aqueuse d'hydrogénocarbonate de sodium à 5 % puis on extrait la phase aqueuse avec 2 fois 20 ml de dichlorométhane. On rassemble les phases organiques, on les sèche sur sulfate de sodium, on les filtre et on évapore.

On reprend le résidu dans 10 ml d'isopropanol et on le traite à 0 °C par 64 ml d'une solution 0,1 N d'acide chlorhydrique dans l'isopropanol. Après évaporation, on décolore le résidu avec du noir animal dans l'acétate d'éthyle et on le purifie sur colonne de Sephadex® LH-20 en éluant par du méthanol. On évapore et on triture le résidu dans l'éther.

On obtient 2 g de produit sous forme d'un solide incolore.

Point de fusion = 130-135 °C Rendement = 51 %

$[\alpha]_D^{20}$ = -112,1 ° (c = 1; chloroforme)

Exemple 4

chlorhydrate de [3a*S*-[2[*R*[*S*(*R*)]], 3aα, 4β, 6β, 7aα]]-[2-[2-[[[4-(5-méthyl-1*H*-imidazol-4-yl)-1-(3a,5,5-triméthylhexahydro-4,6-méthano-1,3,2,-benzodioxaborol-2-yl)butyl]amino] carbonyl]pyrrolidin-1-yl]-2-oxo-1-(phénylméthyl)éthyl] carbamate de 1,1-diméthyléthyle (1:1)

On utilise la méthode décrite dans l'exemple 3, en condensant le chlorhydrate de la [3a*S*[2*R*,3aα,4β,6β,7aα]]-5-méthyl-α-(3a,5,5-triméthylhexahydro-4,6-méthano-1,3,2-benzodioxaborol-2-yl)-1*H*-imidazole-4-butanamine avec l'ester de la *N*-[(1,1-diméthyléthoxy)carbonyl]-*D*-phénylalanyl-2-proline avec la 1-hydroxypyrrolidine-2,5-dione.

On otient 600 mg de produit

Rendement = 28 % PF = 85-90°C

$[\alpha]_D^{20}$ = -53,2 ° (c=0,86 ; méthanol)

## Revendications

1. Composés de formule (I)

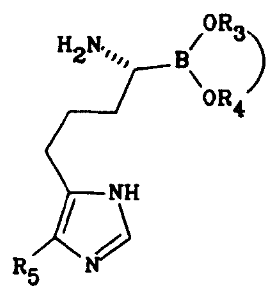

(I)

dans laquelle
$R_3$et $R_4$ représentent ensemble le résidu d'un composé dihydroxylé et
$R_5$ représente un atome d'hydrogène ou un groupe ($C_1$-$C_4$)alkyle sous forme d'isomères optiques ou géométriques, purs ou sous forme de mélanges, ainsi que leurs sels.

2. Composés selon la revendication 1 caractérisés en ce que le composé dihydroxylé est choisi parmi le butane-2,3-diol, le 2,3-diméthylbutane-2,3-diol ou le (1α,3α,5α)-2,6,6-triméthylbicyclo[3.1.1]heptane-2,3-diol [(+)-α-pinanediol] sous forme d'isomères optiques ou géométriques, purs ou sous forme de mélanges, ainsi que leurs sels.

3. Procédé de préparation des composés selon la revendication 1 ou 2, procédé caractérisé en ce qu'on fait réagir le [3a*S*-(3*a*α, 4β, 6β, 7*a*α)]-2-(3-bromopropyl)-3*a*,5,5-triméthylhexahydro-4,6-méthano-1,3,2-benzodioxaborole de formule (IV)

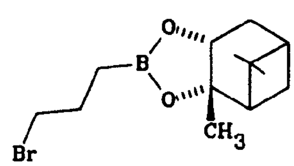

(IV)

avec de l'iodure de sodium dans un solvant aprotique, puis on condense le [3a*S*-(3*a*α, 4β, 6β, 7*a*α)]-2-(3-iodopropyl)-3*a*,5,5-triméthylhexahydro-4,6-méthano-1,3,2-benzodioxaborole de formule (V) ainsi obtenu

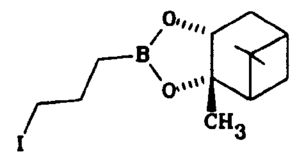

(V)

avec un composé de formule (VI)

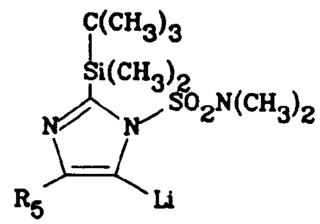

(VI)

(dans laquelle $R_5$ représente un atome d'hydrogène ou un groupe $(C_1\text{-}C_4)$alkyle), pour former un composé de formule (VII)

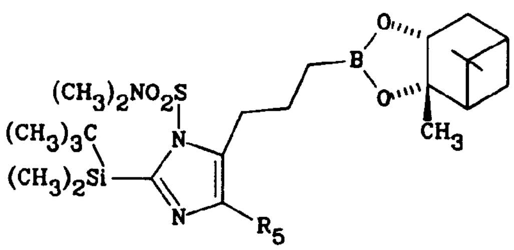

(VII)

que l'on fait réagir avec du dichlorométhyllithium, pour obtenir un composé de formule (VIII)

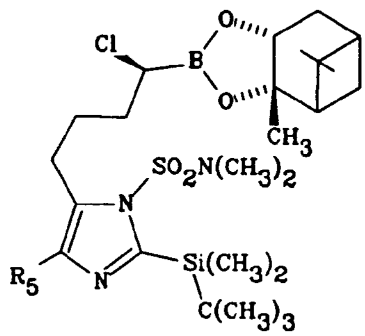

(VIII)

que l'on fait réagir avec du bis(triméthylsilyl)amidure de lithium pour former un composé que l'on traite avec de l'acide chlorhydrique,
pour obtenir un chlorhydrate de formule (IX)

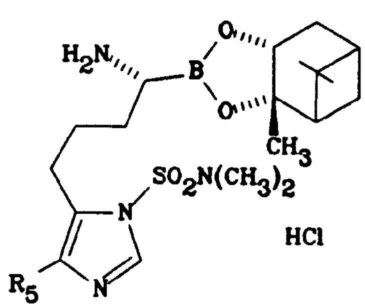

(IX)

que l'on hydrolyse
pour obtenir le dichlorhydrate d'un acide (R)-α-amino-butaneboronique de formule (X)

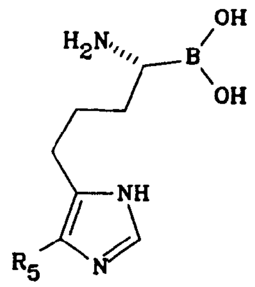

2HCl

(X)

que l'on fait réagir avec un diol de formule (XI)

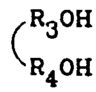

(XI)

dans laquelle $R_3$ et $R_4$ représentent ensemble le résidu d'un composé dihydroxylé tel que défini dans la revendication 1 ou 2 pour obtenir un composé de formule (I).

**4.** Utilisation des composés selon la revendication 1 ou 2 pour la synthèse de composés de formule (1)

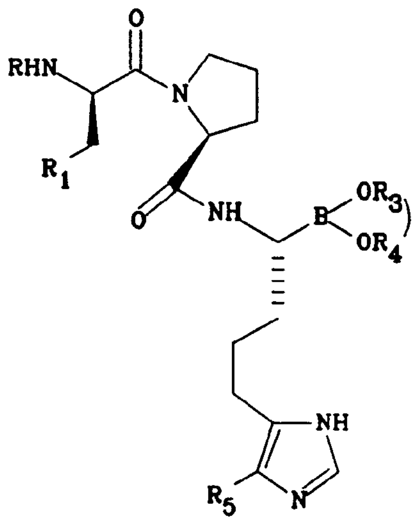

(1)

dans laquelle
R représente soit un atome d'hydrogène, soit un groupe choisi parmi les groupes $(C_1\text{-}C_4)$alkyle droit ou ramifié, -COR' où R' est un groupe $(C_1\text{-}C_4)$alkyle droit ou ramifié et $-(CH_2)_nCO_2R''$ où R" est un atome d'hydrogène ou un groupe $(C_1\text{-}C_4)$alkyle droit ou ramifié ou un groupe phénylméthyle et n = 0 à 2,
$R_1$ représente soit un groupe phényle, soit un groupe naphtalén-1-yle, soit un groupe naphtalén-2-yle, soit un groupe cyclohexyle,
$R_3$ et $R_4$ représentent ensemble le résidu d'un composé dihydroxylé tel que par exemple le butane-2,3-diol, le 2,3-diméthylbutane-2,3-diol ou le (1α, 3α, 5α)-2,6,6-triméthylbicyclo[3.1.1]heptane-2,3-diol [(+)-α-pinanediol] et
$R_5$ représente un atome d'hydrogène ou un groupe $(C_1\text{-}C_4)$alkyle.

Office européen des brevets

# RAPPORT DE RECHERCHE EUROPEENNE

Numero de la demande
EP 95 40 0775

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | US-A-4 537 773 (SHENVI, A.B.)<br>----- | | C07F5/02<br>C07K5/06 |
| | | | **DOMAINES TECHNIQUES RECHERCHES (Int.Cl.6)** |
| | | | C07F<br>C07K |

Le présent rapport a été établi pour toutes les revendications

| Lieu de la recherche | Date d'achèvement de la recherche | Examinateur |
|---|---|---|
| LA HAYE | 3 Juillet 1995 | Rinkel, L |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)